# EUROPEAN PATENT SPECIFICATION

(11) **EP 2 777 749 B1**
(45) Date of publication and mention of the grant of the patent: **09.01.2019**
(21) Application number: 12848488.8
(22) Date of filing: 09.11.2012
(51) Int. Cl.: A61M 25/10, A61M 29/02, A61M 25/00

(54) **BALLOON DILATION CATHETER**
BALLON-DILATATIONSKATHETER
CATHÉTER DE DILATATION À BALLONNET

(30) Priority: 10.11.2011 CN 201110355622
(43) Date of publication of application: 17.09.2014
(73) Proprietor: Microport Endovascular (Shanghai) Co., Ltd, Pudong New District Shanghai 201318 (CN)
(72) Inventor: XIE, Zhiyong, Shanghai 201203 (CN); SUN, Fanghua, Shanghai 201203 (CN); LI, Xiaoxiu, Shanghai 201203 (CN); LU, Huina, Shanghai 201203 (CN); ZHANG, Yingtao, Shanghai 201203 (CN); ZHU, Jiaying, Shanghai 201203 (CN); SONG, Linfei, Shanghai 201203 (CN); GUO, Fang, Shanghai 201203 (CN); LUO, Qiyi, Shanghai 201203 (CN)
(74) Representative: Dantz, Jan Henning
(86) International application number: PCT/CN2012/084406
(87) International publication number: WO 2013/067965

(56) References cited:
- EP-A1- 1 656 963
- EP-A2- 1 121 955
- EP-A2- 1 316 327
- EP-B2- 0 318 918
- WO-A1-2011/130679
- CN-A- 102 139 136
- CN-A- 102 500 041
- US-A- 5 176 661
- US-A- 5 460 608
- US-A1- 2006 129 176
- US-B2- 7 491 213

## Description

### FIEID OF THE INVENTION

The present invention relates to the technical field of medical instruments and, in particular, to a balloon dilation catheter.

### BACKGROUND OF THE INVENTION

The balloon dilation catheter is a tool for angioplasty, and has been widely applied to percutaneous transluminal angioplasty and the clinical medicine of the percutaneous transluminal angioplasty. The balloon dilation catheter is guided by a proper imaging device to be pushed to a position of angiostenosis by means of a percutaneous puncture technique, and the balloon is dilated under close monitoring, so that the position of angiostenosis is dilated to thereby achieve the object of restoring the internal diameter of the lumen and improving the blood circulation.

Most of the existing balloon dilation catheters are composed of polymer materials, and during use, if a long diffuse lesion, in particular a severely calcified lesion, is met, the balloon dilation catheter of a coaxial structure is generally unable to smoothly penetrate the lesion due to an insufficient push force of the catheter, and there even exists a possibility that the catheter is broken in the blood vessel, which influences the operation process and even causes a potential risk to the patient; with respect to the balloon dilation catheter of a fast exchange structure, due to an insufficient push force, it can hardly pass through a long lesion, so the applicable objects are limited. Furthermore, with respect to those balloon dilation catheters of a coaxial structure that directly adopt a whole metallic reinforcing tube, thanks to the wholeness and the comparatively strong anti-bending performance of the metallic reinforcing tubes, they produce a forcibly straightening effect on the blood vessels at the preliminary stage of use, which causes a damage to the blood vessels, and after being used, the balloon dilation catheters usually can hardly be withdrawn due to an irreversible bending deformation of the metallic tube materials and meanwhile cause a secondary damage to the walls of the blood vessels, which not only influences the operation process but also brings a comparatively large mental pressure and pain to the patient.

US 5 460 608 discloses a balloon catheter having an outer shaft and an inner shaft. The inner shaft is reinforced by a stainless steel coil being secured to the outer surface of the inner shaft to prevent it from collapsing or breaking throughout its length and also improving the deflation time of the balloon.

EP 1 121 955 A2 discloses a balloon catheter including an outer tubular member having a proximal end and a distal end. An inflatable balloon is secured to the distal end of the outer tubular member at a proximal portion of the inflatable balloon. The distal portion of the inflatable balloon is secured to the distal end of an inner tubular member. The inner tubular member is comprised of a thin inner layer, a reinforcing layer placed on top of the inner layer and a soft outer layer which surrounds and bounds the reinforcing layer to the inner layer.

In US 5 176 661 a vascular catheter having a tubular member of composite structure with a tubular substrate and a resin-impregnated fibrous covering extending over a substantial part of the length thereof. The composite tubular member is utilized in balloon dilatation catheters used in angioplasty procedures.

EP 1 656 963 A1 discloses an elongate tubular shaft for use in catheter assemblies. Such a catheter assembly for a self-expanding stent comprises an inner shaft located coaxially within an outer shaft. Each of the shafts comprises a metallic hypotube having a plurality of cuts in the side wall thereof to increase the flexibility of the hypotube and to define slots extending about the wall of the hypotube in a substantially spiral or circumferential path which is interrupted at intervals by solid struts.

### SUMMARY OF THE INVENTION

In view of the above, an embodiment of the present invention provides a balloon dilation catheter, in which a reinforcing tube is provided between an outer tube and an inner tube, and the reinforcing tube has a plurality of hollowed-out grooves, to achieve a smooth penetration of a long diffuse lesion location and even a severely calcified lesion location.

To achieve these and in accordance with the purpose of the invention, the embodiment of the present invention provides the following technical solution:
A balloon dilation catheter comprises an outer tube, a reinforcing tube, a balloon, an inner tube and a connector, wherein:
the reinforcing tube runs through an inner chamber of the outer tube, a distal end of the reinforcing tube passes through the balloon, and is sealed with and fixed to a distal end of the balloon, and a plurality of hollowed-out grooves are provided in the reinforcing tube;
the inner tube runs through an inner chamber of the reinforcing tube, and a distal end of the inner tube protrudes from the distal end of the reinforcing tube, and a distal region of the inner tube is sealed with and fixed to the balloon; and
the connector is fixed to a proximal end of the outer tube, and is provided thereon with a first connection port and a second connection port, and the first connection port communicates with a space between an outer surface of the inner tube and an inner surface of the balloon and a space between the outer surface of the inner tube and an inner surface of the outer tube; and the second connection port communicates with a proximal opening of the inner tube.

Preferably, a structure of the reinforcing tube is a columnar structure, and the columnar structure has a cross-sectional area which is constant or gradually reduced from the proximal end to the distal end.

Preferably, the plurality of hollowed-out grooves are annular grooves, and the axes of the plurality of annular grooves coincide with the axis of the outer tube.

Preferably, hollowed-out directions of the plurality of annular grooves are consistent with each other, or hollowed-out directions of two adjacent ones of the plurality of annular grooves are opposite to each other.

Preferably, the width of the annular groove is 0.5-5.0 mm, and the distance between the two adjacent annular grooves is 0.1-10 mm.

Preferably, the circumferential length of the annular groove is 30%∼70% of the circumferential length of the reinforcing tube.

Preferably, the material of the reinforcing tube is a metallic material.

Preferably, the metallic material includes one or more of: stainless steel, Monel alloy, Hastelloy alloy, austenic steel, ferrite steel, martensite steel, nickel, titanium and tantalum.

Preferably, the distal end portion of the inner tube has a bevel angle part which extends for 360 degrees and has a length within a range of 1.00∼4.00 mm.

Preferably, the balloon dilation catheter further comprises at least one radio-opaque marker that is embedded in or sleeved on the inner tube, and the position of the radio-opaque marker corresponds to the position of the balloon.

As can be seen from the above technical solutions, in the balloon dilation catheter provided by the embodiments of the present invention, a reinforcing tube is provided between the outer tube and the inner tube, and a plurality of hollowed-out grooves are provided in the tube body of the reinforcing tube. Since the reinforcing tube is provided, the balloon dilation catheter has a strength, and can smoothly penetrate a long diffuse lesion location and even a severely calcified lesion location during an operation. Meanwhile, since the plurality of hollowed-out grooves enable the reinforcing tube to bend freely, as compared with the existing reinforcing tube formed as a whole tube, the present balloon dilation catheter not only can change a bending angle automatically and flexibly according to the actual bending conditions of the blood vessels to fit with the bent and variable blood vessels to thereby avoid a damage to the blood vessels caused by a forcibly straightening effect on the blood vessels, but also avoids a secondary damage to the blood vessels during withdrawal of the catheter caused by an irreversible bending deformation of the tube material occurring due to an external force, which facilitates proceeding of a vascular dilation operation.

Furthermore, a 360-degree bevel angle processing is performed with respect to the distal end portion of the inner tube located at the distal end of the balloon dilation catheter to reduce the cross-sectional area of the distal end portion of the inner tube, which reduces the resistance suffered during delivery in the blood vessel, and also facilitates the penetration of the blood vessel which is diffuse calcified and even blocked below the knee by the balloon dilation catheter.

### BRIEF DESCRIPTION OF THE DRAWINGS

In order to illustrate the technical solutions in the embodiments of the present invention or the prior art more clearly, the figures to be used in the descriptions of the embodiments or the prior art will be briefly introduced below. It is obvious that the figures in the descriptions below are only some embodiments recorded in the present invention, and those skilled in the art can also obtain other figures according to these figures without making inventive efforts.
Fig. 1 is a structural schematic view of one embodiment of the balloon dilation catheter provided by the present invention;
Fig. 2 is a structural schematic view of one embodiment of the reinforcing tube of the balloon dilation catheter provided by the present invention;
Fig. 3 is a structural schematic view of another embodiment of the reinforcing tube of the balloon dilation catheter provided by the present invention; and
Fig. 4 is a partial structural schematic view of one embodiment of the distal end portion of the inner tube of the balloon dilation catheter provided by the present invention.

### DETAILED DESCRIPTION

In order to make those skilled in the art better understand the technical solutions in the present invention, clear and complete descriptions of the technical solutions in the embodiments of the present invention are performed by taking the figures in the embodiments of the present invention into consideration. It is obvious that the described embodiments are only parts of the embodiments, rather than all of the embodiments, of the present invention. Based on the embodiments in the present invention, all the other embodiments obtained by those skilled in the art without making inventive efforts shall fall within the scope of protection of the present invention.

The expression "distal end" appearing in this text refers to the end close to the patient during delivery, and the expression "proximal end" refers to the end close to the operator during a clinical operation.

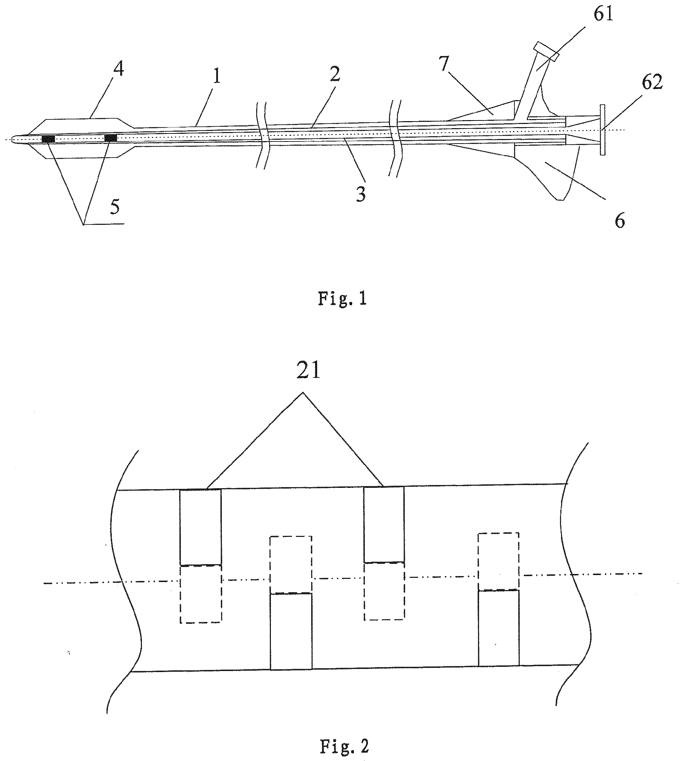

Fig. 1 is a structural schematic view of one embodiment of the balloon dilation catheter provided by the present invention.

As shown in Fig. 1, the balloon dilation catheter comprises an outer tube 1, a reinforcing tube 2, an inner tube 3, a balloon 4 and a connector 6.

The outer tube 1 has a hollow structure, the proximal end of the outer tube 1 is fixed to and sealed with the connector 6, and the distal end of the outer tube 1 is sealed with and fixed to one end of the balloon 4. Furthermore, in order to ensure that the distal end of the outer tube 1 is soft enough to enter the blood vessel and ensure that the proximal end of the outer tube 1 is hard enough to provide a comparatively large push force during pushing, in the other embodiments of the present invention, the outer tube 1 can also adopt a multi-section design, i.e., the hardnesses of the materials of the respective sections composing the outer tube 1 are gradually increased from the distal end to the proximal end of the outer tube 1, e.g., with respect to the materials of the respective sections located at the distal end portion of the outer tube 1, comparatively soft polyamide polymer materials, modified polyamide polymer materials or polyamide polymer composite materials can be selected, and with respect to the materials of the respective sections located at the proximal end portion of the outer tube 1, comparatively hard nylon or mixtures of nylon can be selected.

The balloon 4 is sealed with and fixed to the distal end of the outer tube 1 by thermal welding, laser welding or other manners, and seal is formed at the welding position. In order to make it convenient for the balloon dilation catheter to smoothly pass through a diseased region which suffers many diffuse calcifications and even long blockage, after dilation of the balloon 4, the diameter of the balloon 4 is selected as one within the range of 1.00-6.00 mm, and the length of the balloon 4 is selected as one within the range of 20-300 mm. The selection of these size ranges not only can reduce the damage to the blood vessel during dilation of the balloon 4, but also can make the range of selection of surgical instruments during a balloon dilation operation for treatment be comparatively large. The material of the balloon 4 can be a medical polymer material, e.g., a polyamide polymer material, a modified polyamide polymer material or a polymer composite material. Furthermore, in order to smoothly dilate the blood vessel, the busting pressure that can be borne by the balloon 4 is selected as one within the range of 6-20 atm.

The reinforcing tube 2 runs through the inner chamber of the outer tube 1, and the distal end of the reinforcing tube 2 passes through the balloon 4, and is sealed with and fixed to the other end of the balloon 4. The structure of the reinforcing tube 2 is a columnar structure, and the columnar structure can have a cross-sectional area which is constant from the proximal end to the distal end, i.e., the reinforcing tube 2 has a uniform diameter from its proximal end to its distal end, e.g., a cylindrical structure; furthermore, the columnar structure can also have a cross-sectional area which is gradually reduced from the proximal end to the distal end, e.g., the columnar structure has a diameter being gradually reduced or reduced in the form of a step from the proximal end to the distal end. The reinforcing tube 2 serves the purpose of enhancing the overall strength of the balloon dilation catheter, and enabling the operator to apply a comparatively large push force on the balloon dilation catheter during pushing so that the balloon dilation catheter smoothly passes through the lesion location.

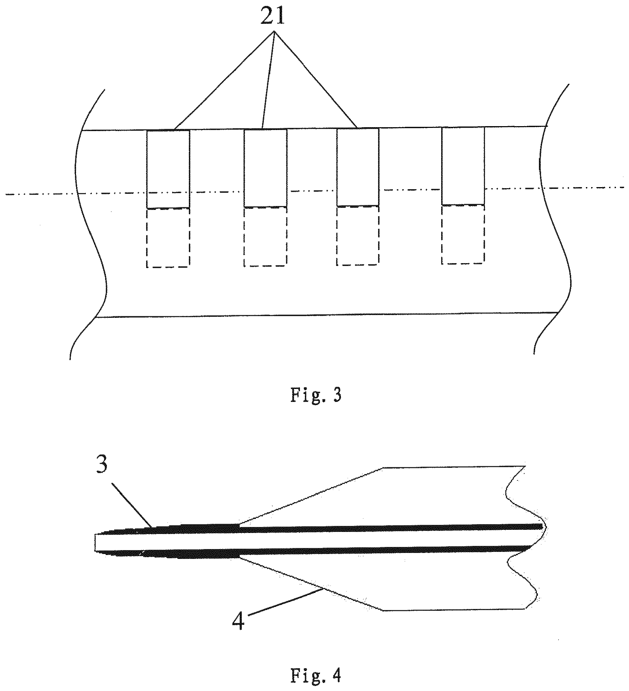

As shown in Fig. 2 and Fig. 3, a plurality of hollowed-out grooves 21 are further provided in the reinforcing tube 2, and since the plurality of hollowed-out grooves 21 are provided in the reinforcing tube 2, when the balloon dilation catheter passes through some bent and variable blood vessels, the reinforcing tube 2 can change a bending angle automatically and flexibly according to the actual bending conditions of the blood vessels to avoid a damage to the blood vessels caused by a forcibly straightening effect on the blood vessels, and meanwhile can further avoid a secondary damage to the blood vessels during withdrawal of the balloon dilation catheter caused by an irreversible bending deformation of the tube material occurring due to an external force, which facilitates proceeding of a vascular dilation operation.

The material of the reinforcing tube 2 can adopt a metallic material, e.g., one or more of common stainless steel, Monel alloy, Hastelloy alloy and other common alloys, or austenic steel, ferrite steel and martensite steel, or nickel, titanium, tantalum and the other metals, and the adoption of the metallic material makes the reinforcing tube 2 have a comparatively large strength and be not easily broken when penetrating the lesion location. Furthermore, in the actual producing process, when the hollowed-out grooves 21 are made in the reinforcing tube 2 made of a metallic material, the hollowed-out grooves 21 can be formed by hollowing out in the reinforcing tube 2 by one or more of the following methods: laser cutting, machinery cutting, gas cutting, water cutting, wire cutting, plasma cutting and the like.

In the embodiments of the present invention, the hollowed-out groove 21 is an annular groove, and the axis of the hollowed-out groove 21 coincides with the axis of the outer tube 1. And in the embodiments of the present invention, the hollowed-out directions of the plurality of hollowed-out grooves 21 provided in the reinforcing tube 2 can be consistent with each other, as shown in Fig. 3; furthermore, the hollowed-out directions of the plurality of hollowed-out grooves 21 can be also freely set according to the desired bending, as shown in Fig. 2, in which the hollowed-out directions of the two adjacent hollowed-out grooves 21 are opposite to each other. In the embodiments of the present invention, the width of each hollowed-out groove 21 can be within 0.5-5.0 mm, the distance between the adjacent hollowed-out grooves 21 can be within 0.1-10 mm, and the circumferential length of the hollowed-out groove 21 can be 30%∼70% of the circumferential length of the reinforcing tube 2.

The inner tube 3 runs through the inner chamber of the reinforcing tube 2, the distal end of the inner tube 3 protrudes from a distal opening of the reinforcing tube 2, and is sealed with and fixed to the balloon 4, the inner tube 3 and the balloon 2 can be also fixed by thermal welding or laser welding, and the welding position is sealed. Since the distal end portion of the inner tube 3 is located at the foremost end of the balloon dilation catheter during penetration of the lesion, in order to reduce the resistance during penetration, as shown in Fig. 4, a polishing processing is performed with respect to the distal end portion of the inner tube 3 located outside the balloon 4 to form a tip having a bevel angle part which extends for 360 degrees, and the length of polishing can be within a range of 1.00-4.00 mm, so during the operation, the distal end portion of the inner tube 3 suffers a comparatively small resistance so as to smoothly pass through the blocked portion in the blood vessel.

As shown in Fig. 1, the connector 6 is fixed to the proximal end of the outer tube 1, and the connector 6 is provided thereon with two connection ports, i.e., a first connection port 61 and a second connection port 62, wherein the first connection port 61 communicates with a space between the outer surface of the inner tube 3 and the inner surface of the balloon 4 and a space between the outer surface of the inner tube 3 and the inner surface of the outer tube 1, and during the operation, when the balloon 4 is delivered to its place, a liquid or gas can be injected into the first connection port 61, so that the balloon 4 is dilated under the action of the pressure to dilate the narrow blood vessel at the lesion; the second connection port 62 communicates with a proximal opening of the inner tube 3, and during the operation, the leading end of the guide wire can be firstly penetrated into the blood vessel, the tailing end of the guide wire is then penetrated from the distal opening of the inner tube 3 and out of the second connection port 62, so that the balloon dilation catheter can be delivered to its place under the guide of the guide wire.

Furthermore, in the other embodiments of the present invention, as shown in Fig. 1, the balloon dilation catheter can further comprise at least one radio-opaque marker 5 that is embedded in or sleeved on the inner tube 3, and the position of the radio-opaque marker 5 corresponds to the position of the balloon 4 for displaying the position of the balloon 4 during pushing. In the embodiments of the present invention, the number of the radio-opaque markers 5 is two, and the two radio-opaque markers 5 are respectively provided on the positions of the inner tube 3 corresponding to both ends of the balloon 4.

In order to prevent the proximal end of the balloon dilation catheter from being broken during pushing, in the other embodiments of the present invention, the balloon dilation catheter can further comprise a stress diffusion tube 7 which is fixed between the outer tube 1 and the connector 6 or encloses and covers the connection part of the outer tube 1 and the connector 6, and which serves the purpose of enhancing the strength between the proximal end of the outer tube 1 and the connector 6.

## Claims

1. A balloon dilation catheter comprising :
an outer tube (1), a reinforcing tube (2), a balloon (4), an inner tube (3) and a connector (6), wherein:
the reinforcing tube (2) runs through an inner chamber of the outer tube (1), a distal end of the reinforcing tube (2) passes through the balloon (4), and is sealed with and fixed to a distal end of the balloon (4), and a plurality of hollowed-out grooves are provided in the reinforcing tube (2);
the inner tube (3) runs through an inner chamber of the reinforcing tube (2), and a distal end of the inner tube (3) protrudes from the distal end of the reinforcing tube (2), and a distal region of the inner tube (3) is sealed with and fixed to the balloon (4); and the connector (6) is fixed to a proximal end of the outer tube (1), and is provided thereon with a first connection port (61) and a second connection port (62), and the first connection port (61) communicates with a space between an outer surface of the inner tube (3) and an inner surface of the balloon (4), and a space between the outer surface of the inner tube (3) and an inner surface of the outer tube (1); and the second connection port (62) communicates with a proximal opening of the inner tube (3).

2. The balloon dilation catheter according to claim 1, **characterized in that** a structure of the reinforcing tube (2) is a columnar structure, and the columnar structure has a cross-sectional area which is constant or gradually reduced from the proximal end to the distal end.

3. The balloon dilation catheter according to claim 1, **characterized in that** the plurality of hollowed-out grooves are annular grooves, and axes of the plurality of annular grooves coincide with an axis of the outer tube (1).

4. The balloon dilation catheter according to claim 3, **characterized in that** hollowed-out directions of the plurality of annular grooves are consistent with each other, or hollowed-out directions of two adjacent ones of the plurality of annular grooves are opposite to each other.

5. The balloon dilation catheter according to claim 3, **characterized in that** a width of the annular groove is 0.5-5.0 mm, and a distance between the two adjacent annular grooves is 0.1-10 mm.

6. The balloon dilation catheter according to claim 3, **characterized in that** a circumferential length of the annular groove is 30%∼70% of a circumferential length of the reinforcing tube (2).

7. The balloon dilation catheter according to claim 1, **characterized in that** the material of the reinforcing tube (2) is a metallic material.

8. The balloon dilation catheter according to claim 7, **characterized in that** the metallic material includes one or more of: stainless steel, Monel alloy, austenic steel, ferrite steel, martensite steel, nickel, titanium and tantalum.

9. The balloon dilation catheter according to claim 1, **characterized in that** the distal end portion of the inner tube (3) has a bevel angle part which extends for 360 degrees and has a length within a range of 1.00∼4.00 mm.

10. The balloon dilation catheter according to claim 1, **characterized by** further comprising at least one radio-opaque marker that is embedded in or sleeved on the inner tube (3), and a position of the radio-opaque marker corresponds to a position of the balloon (4).

## Patentansprüche

1. Ballondilatationskatheter, umfassend:
ein äußeres Rohr (1), ein Verstärkungsrohr (2), einen Ballon (4), ein inneres Rohr (3) und einen Verbinder (6), wobei:
das Verstärkungsrohr (2) durch eine innere Kammer des äußeren Rohrs (1) verläuft, ein distales Ende des Verstärkungsrohrs (2) den Ballon (4) passiert und mit einem distalen Ende des Ballons (4) versiegelt ist und an diesem befestigt ist, und eine Vielzahl von ausgehöhlten Nuten in dem Verstärkungsrohr (2) vorgesehen sind;
wobei das innere Rohr (3) durch eine innere Kammer des Verstärkungsrohrs (2) verläuft und ein distales Ende des inneren Rohrs (3) vom distalen Ende des Verstärkungsrohrs (2) vorragt, und wobei ein distaler Bereich des inneren Rohrs (3) mit dem Ballon (4) versiegelt und an diesem befestigt ist; und wobei der Verbinder (6) an einem proximalen Ende des äußeren Rohrs (1) befestigt ist und mit einem ersten Verbindungsanschluss (61) und einem zweiten Verbindungsanschluss (62) daran vorgesehen ist, und wobei der erste Verbindungsanschluss (61) mit einem Raum zwischen einer äußeren Oberfläche des inneren Rohrs (3) und einer inneren Oberfläche des Ballons (4) und einem Raum zwischen der äußeren Oberfläche des inneren Rohrs (3) und einer inneren Oberfläche des äußeren Rohrs (1) kommuniziert; und wobei der zweite Verbindungsanschluss (62) mit einer proximalen Öffnung des inneren Rohrs (3) kommuniziert.

2. Ballondilatationskatheter nach Anspruch 1, **dadurch gekennzeichnet, dass** eine Struktur des Verstärkungsrohrs (2) eine säulenförmige Struktur ist und die säulenförmige Struktur eine Querschnittsfläche aufweist, die konstant ist oder vom proximalen Ende zum distalen Ende allmählich kleiner wird.

3. Ballondilatationskatheter nach Anspruch 1, **dadurch gekennzeichnet, dass** die Vielzahl von ausgehöhlten Nuten ringförmige Nuten sind und Achsen der Vielzahl von ringförmigen Nuten mit einer Achse des äußeren Rohrs (1) zusammenfallen.

4. Ballondilatationskatheter nach Anspruch 3, **dadurch gekennzeichnet, dass** ausgehöhlte Richtungen der Vielzahl von ringförmigen Nuten miteinander konsistent sind oder ausgehöhlte Richtungen von zwei benachbarten der Vielzahl von ringförmigen Nuten einander entgegengesetzt sind.

5. Ballondilatationskatheter nach Anspruch 3, **dadurch gekennzeichnet, dass** eine Breite der ringförmigen Nut 0,5 bis 5,0 mm beträgt und ein Abstand zwischen den zwei benachbarten ringförmigen Nuten 0,1 bis 10 mm beträgt.

6. Ballondilatationskatheter nach Anspruch 3, **dadurch gekennzeichnet, dass** eine Umfangslänge der ringförmigen Nut 30 % bis 70 % einer Umfangslänge des Verstärkungsrohrs (2) beträgt.

7. Ballondilatationskatheter nach Anspruch 1, **dadurch gekennzeichnet, dass** das Material des Verstärkungsrohrs (2) ein metallisches Material ist.

8. Ballondilatationskatheter nach Anspruch 7, **dadurch gekennzeichnet, dass** das metallische Material eines oder mehreres von Folgendem beinhaltet: rostfreier Stahl, Monel-Legierung, Austenitstahl, Ferritstahl, Martensitstahl, Nickel, Titan und Tantal.

9. Ballondilatationskatheter nach Anspruch 1, **dadurch gekennzeichnet, dass** der distale Endabschnitt des inneren Rohrs (3) einen Abschrägungswinkelteil aufweist, der sich über 360 Grad erstreckt und eine Länge in einem Bereich von 1,00 bis 4,00 mm aufweist.

10. Ballondilatationskatheter nach Anspruch 1, **dadurch gekennzeichnet, dass** er ferner wenigstens eine strahlenundurchlässige Markierung umfasst, die in dem inneren Rohr (3) eingebettet ist oder darauf aufgeschoben ist, und eine Position der strahlenundurchlässigen Markierung einer Position des Ballons (4) entspricht.

## Revendications

1. Cathéter à ballonnet de dilatation comprenant :
un tube externe (1), un tube de renfort (2), un ballonnet (4), un tube interne (3) et un raccord (6), dans lequel :
le tube de renfort (2) court à travers une chambre interne du tube externe (1), une extrémité distale du tube de renfort (2) passe à travers le ballonnet (4), et est obturée avec et fixée à une extrémité distale du ballonnet (4), et une pluralité de rainures évidées sont ménagées dans le tube de renfort (2) ;
le tube interne (3) court à travers une chambre interne du tube de renfort (2), et une extrémité distale du tube interne (3) dépasse de l'extrémité distale du tube de renfort (2), et une région distale du tube interne (3) est obturée avec et fixée au ballonnet (4) ; et
le raccord (6) est fixé à une extrémité proximale du tube externe (1), et est doté, dessus, d'un premier orifice de raccordement (61) et d'un second orifice de raccordement (62), et le premier orifice de raccordement (61) communique avec un espace entre une surface externe du tube interne (3) et une surface interne du ballonnet (4), et un espace entre la surface externe du tube interne (3) et une surface interne du tube externe (1) ; et le second orifice de raccordement (62) communique avec une ouverture proximale du tube interne (3).

2. Cathéter à ballonnet de dilatation selon la revendication 1, **caractérisé en ce qu'**une structure du tube de renfort (2) est une structure colonnaire, et la structure colonnaire a une aire en coupe droite qui est constante ou progressivement réduite de l'extrémité proximale à l'extrémité distale.

3. Cathéter à ballonnet de dilatation selon la revendication 1, **caractérisé en ce que** la pluralité de rainures évidées sont des rainures annulaires, et des axes de la pluralité de rainures annulaires coïncident avec un axe du tube externe (1).

4. Cathéter à ballonnet de dilatation selon la revendication 3, **caractérisé en ce que** les directions évidées de la pluralité de rainures annulaires sont cohérentes les unes avec les autres, ou des directions évidées de deux rainures adjacentes de la pluralité de rainures annulaires sont opposées l'une à l'autre.

5. Cathéter à ballonnet de dilatation selon la revendication 3, **caractérisé en ce qu'**une largeur de la rainure annulaire est de 0,5 à 5,0 mm, et une distance entre les deux rainures annulaires adjacentes est de 0,1 à 10 mm.

6. Cathéter à ballonnet de dilatation selon la revendication 3, **caractérisé en ce qu'**une longueur circonférentielle de la rainure annulaire est de 30 % à 70 % d'une longueur circonférentielle du tube de renfort (2).

7. Cathéter à ballonnet de dilatation selon la revendication 1, **caractérisé en ce que** la matière du tube de renfort (2) est une matière métallique.

8. Cathéter à ballonnet de dilatation selon la revendication 7, **caractérisé en ce que** la matière métallique inclut un ou plusieurs parmi : l'acier inoxydable, l'alliage Monel, l'acier austénique, l'acier ferritique, l'acier martensitique, le nickel, le titane et le tantale.

9. Cathéter à ballonnet de dilatation selon la revendication 1, **caractérisé en ce que** la portion d'extrémité distale du tube interne (3) a une partie à angle de biseau qui s'étend sur 360 degrés et a une longueur dans une plage de 1,00 à 4,00 mm.

10. Cathéter à ballonnet de dilatation selon la revendication 1, **caractérisé en ce qu'**il comprend en outre au moins un marqueur radio-opaque qui est noyé dans ou emmanché sur le tube interne (3), et une position du marqueur radio-opaque correspond à une position du ballonnet (4).
